# EUROPEAN PATENT APPLICATION

(11) **EP 3 450 565 A1**
(43) Date of publication of application: **06.03.2019**
(21) Application number: 17189514.7
(22) Date of filing: 05.09.2017
(51) Int. Cl.: C12P 7/62, C12M 1/107, C12P 7/64, C12P 13/02

(54) **METHOD AND APPARATUS FOR PRODUCING ESTERS OR AMIDES WITH IN SITU PRODUCT RECOVERY**

(71) Applicant: VITO NV, 2400 Mol (BE)
(72) Inventor: VAN HECKE, Wouter, B-2400 Mol (BE); ELSLANDER, Helmut, B-2400 Mol (BE); VANBROEKHOVEN, Karolien, B-2400 Mol (BE)
(74) Representative: Pronovem

(57) **Abstract**

Method of forming an ester or an amide and corresponding apparatus. The method comprises adding a substrate (14) to a reaction liquid (132) comprised in a reactor (13, 33), and reacting the substrate in the reactor to form a product being the ester or the amide. A first stream (15) is separated from the reaction liquid through a first membrane (115) and the product is recovered from the first stream. A second stream (16), including a compound distinct from the product, is separated from the reaction liquid through a second membrane (125). The first membrane and the second membrane are moved relative to the reactor during the separation steps.

## Description

### Technical field

The present invention is related to methods and apparatuses for producing esters or amides. In particular, the present invention is related to methods and apparatuses in which reaction products including the ester or the amide, and water or an alcohol are (continuously) withdrawn from the reactor.

### Background art

The production of esters or amides often involves producing water or an alcohol such as methanol as a by-product. Since these reactions are equilibrium reactions, removing the by-products will shift the equilibrium to the right hand side, increasing product yield. Various options exist for withdrawing the by-products water and/or alcohol, such as using desiccants (e.g., molecular sieves), using a stripping gas, or by pervaporation. The use of desiccants is problematic during attempts of process upscaling, since they tend to pulverize, or cause problems in the downstream processes. The stripping gas in most cases entrains a large part of any volatile compounds present in the reactor.

The use of pervaporation for removing water and/or alcohol appears promising, but requires a profound control of process conditions. In a pervaporation unit, temperature and concentration gradients must be minimized, otherwise leading to losses in flux. To avoid these problems, a high degree of cross-flow is applied in order to minimize these gradients. This however requires a high energy input.

### Summary of the invention

It is therefore an aim of the present description to provide methods and apparatuses which overcome the above drawbacks. In particular, it is an aim to provide methods and apparatuses which are more efficient in producing esters or amides. It is furthermore an aim of the present description to provide methods and apparatuses which have a reduced complexity and/or allow for simplifying downstream processing.

According to a first aspect of the invention, there is therefore provided a method of forming an ester or an amide as set out in the appended claims. The method comprises adding a substrate to a reaction liquid comprised in a reactor and reacting the substrate in the reactor to form a product being the ester or the amide. Advantageously during the reacting step, a first stream is separated or withdrawn from the reaction liquid through a first membrane, advantageously a semipermeable membrane, and the product is advantageously recovered from the first stream. Advantageously, a catalyst is mixed with the reaction liquid in the reactor, and the first membrane is configured for retaining the catalyst. Advantageously simultaneously with separating the first stream, a second stream is separated or withdrawn from the reaction liquid through a second membrane, advantageously a semipermeable membrane. The second stream advantageously comprises a compound distinct from the product. The first stream and the second stream are withdrawn separately from the reactor. According to an aspect, either one or both of the first membrane and the second membrane, which are advantageously (at least partially) immersed in the reaction liquid, are moved relative to the reactor during the steps of separating the first stream and separating the second stream. Advantageously, the above separation steps are carried out in parallel, e.g. simultaneously. The first membrane is advantageously a filtration membrane, e.g. a microfiltration or ultrafiltration membrane. The second membrane is advantageously a pervaporation membrane, or a contactor membrane.

According to a second aspect of the invention, there is provided an apparatus as set out in the appended claims. The apparatus may be configured to carry out a method according to the above aspects. The apparatus comprises a reactor, a first membrane and a second membrane. The reactor comprises a reactor vessel, a substrate supply port, and either one or both of a first outlet port and a second outlet port. The first membrane has a first surface communicating with the reactor vessel and a second surface, opposite the first surface, communicating with the first outlet port. The second membrane has a third surface communicating with the reactor vessel and a fourth surface, opposite the third surface, communicating with the second outlet port. The first membrane is advantageously configured for transport of one or more compounds from the first surface to the second surface, e.g. the first membrane is a semipermeable membrane. The second membrane is advantageously configured for transport of one or more compounds from the third surface to the fourth surface, e.g. the second membrane is a semipermeable membrane. According to an aspect, either one or both of the first membrane and the second membrane is moveably arranged relative to the reactor vessel. Advantageously, the first surface and the third surface are accessible in parallel from the reactor vessel. Advantageously, the first membrane and the second membrane are mounted in the reactor vessel such that they are immersed in a reaction liquid when in use.

By moving the membranes relative to the reactor, less pumping energy is required for sustaining a desired cross flow over the membrane surface. Furthermore, by appropriately maintaining the reaction liquid in motion relative to the membranes simplifies the requirements for substrate supply control, such as for temperature and concentration gradient control. Temperature gradients are non-existing or greatly minimized, and concentration gradients over a surface of the membranes are non-existing or easily minimized. A further advantage of the apparatuses and processes described herein is that they are inherently safer because no associated (external) loops with high cross flows are involved. There is no or less external piping which may be prone to leakage and/or which may expose maintenance operators to (potentially) toxic products. A further advantage is that they allow for integrating the membranes within the reactor vessel resulting in reactors with small footprints, taking much less space in comparison to prior art designs.

### Brief description of the figures

Aspects of the invention will now be described in more detail with reference to the appended drawings, wherein same reference numerals illustrate same features and wherein:
Figure 1 represents schematically an apparatus integrating two separate membrane units in the reactor according to aspects described herein;
Figures 2A-B represent a horizontal respectively vertical cross section of a reactor according to aspects described herein, wherein two membrane units are mounted on rotating supports within the reactor vessel; Fig. 2B represents a cross section view along section line A-A;
Figure 3 represents a (horizontal) cross section of an alternative reactor compared to Fig. 2A, wherein the two membrane units are mounted on a single rotating support within the reactor vessel.

### Description of embodiments

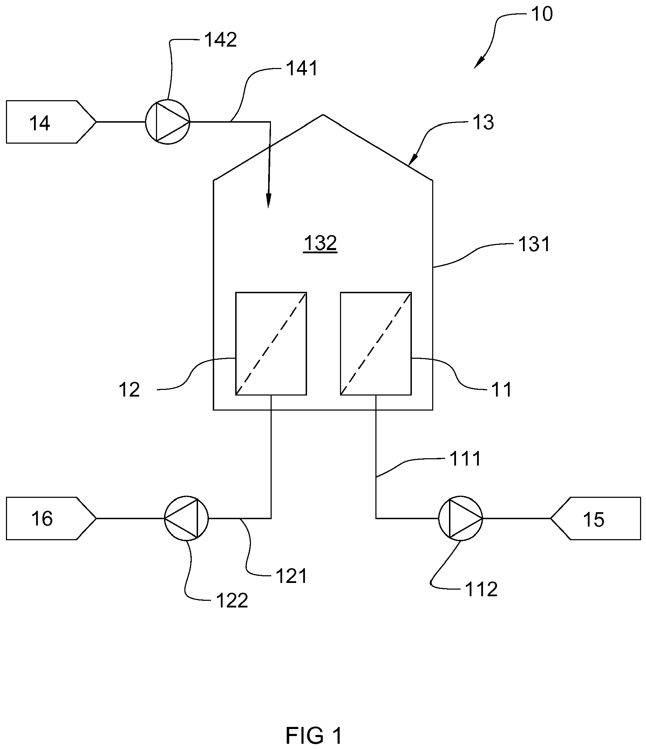

Referring to Fig. 1, an apparatus 10 according to aspects described herein, integrates two separate, distinct membrane units 11 and 12 within a reactor 13. Reactor 13 is advantageously a reactor for conversion of a substrate 14, in particular a biomass substrate, to a useful product such as an ester or amide, in the presence of a catalyst, advantageously a biocatalyst, such as microorganisms or enzymes. Reactor 13 comprises a reactor vessel 131 containing a reaction liquid 132 including (partially) converted and non-converted feedstock, and optionally the catalyst. Substrate 14 is advantageously continuously supplied to the reactor vessel 131 of reactor 13 through a supply duct 141 via a pump 142. The substrate 14 advantageously comprises substrate compounds for the catalyst for carrying out the conversion reactions.

The catalyst is advantageously housed in the reactor 13 (reactor vessel 131), and may or may not be immobilised therein. The catalyst is advantageously mobile in the reactor 13, e.g. it is mixed or suspended in the reaction liquid. Reaction products are advantageously continuously or intermittently (e.g., after depletion of a substrate) removed from the reactor 13 through membrane units 11 and 12. Each of the membrane units 11 and 12 is advantageously configured to recover or withdraw specific reaction products. Either one, or both the membrane units 11 and 12 advantageously allow for retaining the catalyst in the reactor 13.

A first membrane unit 11 may be configured to withdraw a first stream 15 from reactor 13. Membrane unit 11 advantageously comprises semipermeable membranes for producing the first stream 15 as a permeate stream from the reaction liquid 132 in the reactor 13. By way of example, membrane unit 11 is configured as a filtration unit, comprising filtration membranes. Membrane unit 11 is coupled to an outlet duct 111 at the permeate side. A pump 112 in communication with the outlet duct 111 may be configured for installing a desired pressure difference across the membranes of membrane unit 11.

The first stream (permeate) 15 advantageously comprises, or consists of a desired product to be recovered, such as an ester or an amide compound. The desired product may be formed through conversion processes occurring in the reactor 13, e.g. esterification and amidation reactions. Additional compounds present in reaction liquid 132 may permeate through the membrane unit. The desired product may be recovered from the first stream 15 through appropriate downstream processing, e.g. by distillation. Unconverted substrates may permeate through the membrane unit 11. These permeated substrates can be recycled to the reactor 13 after separation from the product to improve substrate utilization.

The membranes of membrane unit 11 are advantageously filtration membranes. Specific examples of suitable filtration membranes are microfiltration and ultrafiltration membranes. Another example of useful filtration membranes are nanofiltration membranes.

A second membrane unit 12 may be configured to withdraw a second stream 16 from reactor 13. The second membrane unit 12 is advantageously configured as a pervaporation unit. The second membrane unit 12 advantageously comprises semipermeable membranes. The semipermeable membranes are advantageously suitable for use in pervaporation processes. A pervaporation unit separates the second stream 16 from the reaction liquid 132 through permeation and evaporation of the second stream 16. Alternatively, the second membrane unit 12 may be configured for liquid-liquid extraction, e.g. as a membrane contactor. In this case, the second membrane unit 12 comprises contactor membranes, and a liquid solution is made to circulate at the permeate side of the membrane. A difference in solubility or a concentration gradient provides the driving force to generate a mass transfer of selective compounds across the membranes, hence forming the second stream 16. The second membrane unit 12 is coupled to an outlet duct 121 at the vapour side of the unit. A pump, in particular a vacuum pump 122 (in case of pervaporation) may communicate with the outlet duct 121 and/or the vapour side of the second membrane unit 12 to maintain a desired (vacuum or partial vacuum) pressure level, or a desired flow rate (in case of membrane contactor).

The second stream 16 may comprise or consist of a by-product of conversion reactions occurring in the reactor 13. The by-product may be an inhibitory compound which is formed from the conversion reactions, such as water and/or an alcohol, e.g. methanol. An inhibitory compound refers to a compound which prevents further conversion of the substrate to the desired product to take place in reactor 13, in particular because of an equilibrium being reached between the reagents, e.g. the substrate, and the reaction products. By removing the inhibitory compound, a rate of formation of the desired products can be increased. Furthermore, by removing the inhibitory compound, the catalyst may be protected from inactivation.

According to an aspect, the membranes of the first membrane unit 11 and the membranes of the second membrane unit 12 are arranged inside the reactor 13. The membranes are advantageously at least partially and preferably fully immersed in the reaction liquid of the reactor. In particular, these membranes are arranged inside the vessel 131 of the reactor.

A pervaporation or membrane contactor unit integrated within the reactor vessel simplifies the requirements for temperature and concentration gradient control. Temperature gradients are minimized, and concentration gradients over a surface of the membranes can easily be minimized by appropriately maintaining the reaction liquid 132 in motion relative to the second membrane unit 12. A same reasoning may apply to a membrane filtration unit, such as the first membrane unit 11.

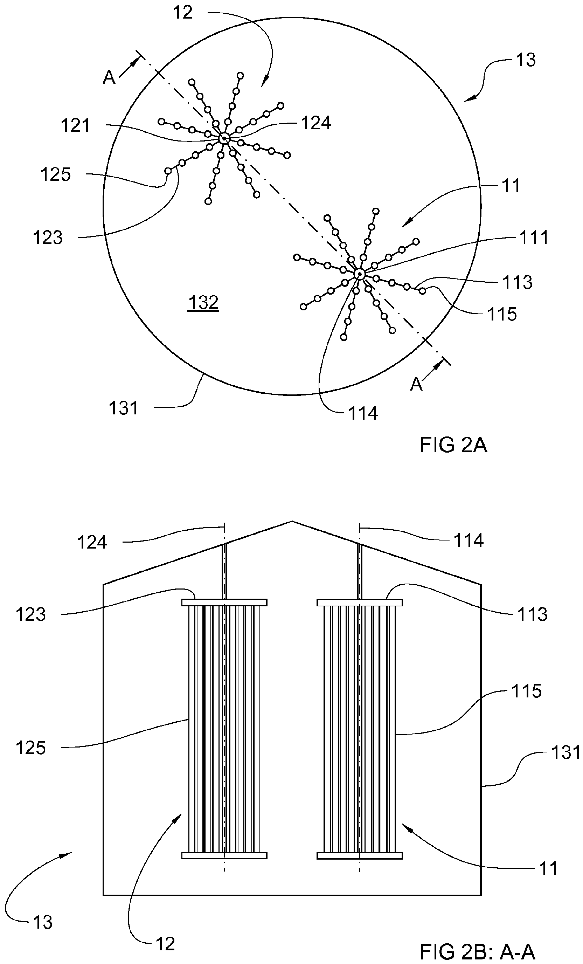

According to an aspect, and referring to Figs. 2A-B, the membranes of units 11 and 12 are moveably arranged within the reactor 13. In particular, the membranes of units 11 and 12 are arranged to move relative to the reactor 13 (reactor vessel 131). Each of the first and second membrane units 11, 12 may comprise a support frame 113, 123 respectively which is pivotally arranged relative to reactor vessel 131. Support frames 113, 123 may be configured to turn on pivot axes 114, 124 fixed to the reactor vessel 131, such as through a rotating or oscillating motion.

Respective membranes 115, 125 of the first and second membrane units 11, 12 are mounted to the respective support frames 113, 123. The membranes are advantageously fixedly attached to the respective support frames. Each of the membranes 115, 125 comprise a first, outer surface in (direct) contact with, and advantageously immersed in the reaction liquid 132, and an opposite, second (inner) surface which communicates with the respective outlet duct 111, 121, e.g. through an appropriate manifold, which may be integrated in frame 113, 123. The membrane 115, 125 advantageously provides for transport of compounds from the outer surface to the inner surface, while other compounds may be retained or rejected at the outer surface. The membranes 115, 125 are advantageously hollow fibre membranes or tubular membranes, even though planar membranes, so called flat sheet membranes, and which may be arranged flat (e.g. to act like impellers) or may be spirally wound, can be used as well. The support frames 113, 123 and the disposition of the membranes 115, 125 thereon may have any desired configuration. Specific, non-limiting examples of such a configuration are support frames formed as radial brackets (as shown in Fig. 2A), concentric rings, brackets winding spirally about the pivot axis, etc.

When the support frames 113, 123 with the membranes 115, 125 are turned on axes 114, 124, the reaction liquid is advantageously mixed. The support frames and membranes therefore may be used in replacement of, or additional to impellers in the reactor. This minimizes concentration and temperature gradients in the vessel, minimizes concentration polarization effects on the surface of the membranes and/or reduces fouling of the membranes 115, 125. The motion of support frames 113, 123 may be continuous or intermittent. Specific examples of this motion are a continuous rotation on axes 114, 124, and an oscillating motion, e.g. back and forth on axes 114, 124, advantageously over an angle ranging between 120° and 240°, e.g. 180°. An actuator, such as an electric motor, possibly arranged outside the reactor vessel 131, may be coupled to the axis 114 and/or 124 to provide the pivoting motion.

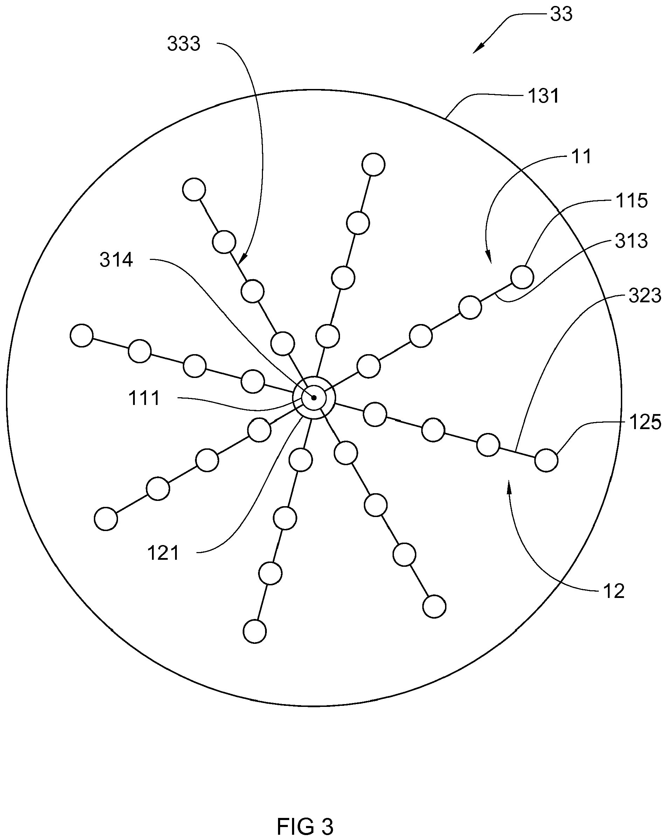

Fig. 3 shows an alternative configuration of a reactor 33. Reactor 33 differs from reactor 13 in that the membrane units 11, 12 are mounted on a common support frame 333, which is pivotally arranged on axis 314 fixed with respect to reactor vessel 131. Support frame 333 advantageously comprises first brackets 313 onto which the membranes 115 relating to the first membrane unit 11 are mounted, and second brackets 323 onto which the membranes 125 relating to the second membrane unit 12 are mounted. The first and second brackets may be disposed as desired on pivot axis 314. By way of example, the first and second brackets may be disposed alternatingly about axis 314. Each one bracket 313, 323 advantageously comprises a collector manifold for collecting the respective first and second streams 15, 16 from the membranes, and communicating with the corresponding outlet duct 111, 121.

As with reactor 13, the support frame 333 of reactor 33 may be configured to turn on axis 314 with any desired motion, e.g. (continuous) rotation or oscillation. The shape of the support frame is not particularly limited, and any suitable shape and/or disposition of the brackets 313, 323 may be used. Possible configurations for the support frame and which can be used in the reactors described herein, are described in US 2009/0034358 to Brod et al. and US 8328167 to Kauling et al. It should be noted that in the above documents, the rotating membrane units are configured for gassing of liquids, whereas in the present description the rotating membrane units are configured for withdrawing two distinct streams from the reaction liquid.

The reactor configurations described above in relation to Figs. 1-3 are advantageously used for processes involving forming esters or amides.

According to one aspect, the above reactor configurations are used in a process of forming an ester, in particular a chiral ester. Different methods for forming esters exist, and all of them are in principle contemplated in the present description. One method of forming esters involves direct esterification. An alcohol and an organic acid, e.g. a carboxylic acid, are used as substrates and made to react to form the ester and water. Another method involves transesterification. An alcohol and an ester, e.g. methyl ester, are used as substrates and are made to react to form the ester and an alcohol, e.g. methanol. Other possible methods of forming esters involve alcoholysis of acyl chlorides or acid anhydrides.

Methods according to aspects described herein are particularly interesting for ester production where one and/or both substrates are volatile and/or forms azeotropes with methanol or water. In such cases, nitrogen stripping is rendered complicated due to downstream process complications. Examples of useful esters that may be produced in methods described herein are: isopropyl esters, methacrylate esters and acrylate esters.

According to another aspect, the above reactor configurations are used in a process of forming an amide, in particular a chiral amide, in particular an organic amide. Different methods for forming amides exist, and all of them are in principle contemplated in the present description. In one method of forming amides, an advantageously organic amine and an organic acid, e.g. a carboxylic acid, are used as substrates and made to react to form an (organic) amide and water. Other possible methods involve using acid anhydrides or acyl chlorides.

Methods according to aspects described herein are particularly interesting for production of amides where one of and/or both the substrates are volatile and/or form azeotropes with methanol/water. In such cases, nitrogen stripping is rendered complicated due to downstream process complications. Examples of useful processes include amide production processes where isopropyl amine or n-propyl amine is used as substrate, or where (meth)acrylic acid, or (meth)acrylate esters are used as substrates.

The substrates are supplied to reactor 13 or 33. Substrate pump 142 may be configured to supply substrates in controlled amounts to the reactor. Substrates may be pre-mixed prior to being supplied to the reactor, or they may be fed separately to the reactor. A catalyst may be provided in the reactor, either immobilized or freely suspended in the reactor. The catalyst is advantageously a biocatalyst, and processes described herein are advantageously biocatalytic processes. The (bio)catalyst advantageously comprises or consists of an enzyme, such as a lipase or an esterase. Examples of useful catalysts include lipases from *Candida antarctica,* from *Burkholderia* species, from *Fervidobacterium nodosum,* from *Starmerella bombicola.* A commercial name of an immobilised lipase is Novozym® 435 (Novozymes, Denmark) which is immobilised on acrylic resin. The enzyme, such as the lipase, may be freely suspended in the reactor or may be immobilized on a carrier. The (bio)catalyst may be a microorganism which may catalyze the reactions through an enzyme contained therein.

The substrates, e.g. supplied through stream 14, react in the reactor, possibly in the presence of the (bio)catalyst, to form the ester or the amide, and water or an alcohol, which build up in the reaction liquid 132. Reaction conditions in the reactor are advantageously mild, and temperature of the (reaction liquid in the) reactor may be maintained at between 30°C and 70°C, advantageously between 40°C and 65°C, advantageously between 50°C and 60°C. Advantageously, no (organic) solvents are used in the reaction liquid 132 to form the ester or the amide.

The support frames 113, 123 of the first and second membrane units 11, 12, or the support frame 333 placed inside the reactor vessel 131, are advantageously moved relative to the reactor, e.g. by imparting a pivoting motion on axis 114, 124 or 314 oscillating back and forth over a predetermined angle, which may range between 120° and 240°, e.g. 180° or less.

The membranes 115, 125 mounted on the respective support frame are in contact with the reaction liquid, e.g. they are immersed in the reaction liquid. As the membranes move integrally with the support frame, a relative motion between the reaction liquid and the membranes is sustained. In the examples of Figs. 2A-B and 3, the (hollow fibre or tubular) membranes have longitudinal axes oriented parallel to the pivot axes 114, 124, 314, and therefore are oriented perpendicular to the plane of motion. These configurations may be effective in maintaining a sufficient level of cross flow of the reaction liquid over the external surface of the membranes.

In the first membrane unit 11 a suitable pressure difference across the membranes 115 (the transmembrane pressure) is advantageously applied, e.g. via pump 112. This will withdraw a first stream (permeate) from the reaction liquid which is collected in permeate channels (e.g., the internal lumens of membranes 115) of the membrane unit 11 and further evacuated via a collector manifold to the outlet duct 111. Through suitable selection of the type of membrane 115 (e.g., microfiltration, ultrafiltration, etc.), specific compounds may be selectively recovered from the reaction liquid, e.g. on the basis of molecule size. These compounds advantageously comprise the ester or the amide that is obtained through reaction in the reactor. Possibly, a portion of the substrate(s) may pass through the membranes 115. Membranes 115 may be selected to block passage of the (bio)catalyst, which will be retained in the reactor.

It will be convenient to note that filtration membranes are just one exemplary type of membranes that can be used in the apparatuses and processes described herein. Other possible membranes may be configured to entrain a selective mass transfer across the membranes via mechanisms other than differential pressure and/or characteristic pore size, such as though not limited to: selective sorption, or solution or diffusion characteristics, e.g. membrane contactors.

The permeate collected by the membranes 115 forms a first stream 15, which may be further processed via an appropriate downstream processing to separate the different compounds from which it is composed. The desired ester or amide can e.g. be recovered from stream 15 by distillation. Other compounds (e.g. the substrates) may be recovered, and may be recycled to the reactor 13, 33.

The membranes 125 of the second membrane unit 12 will withdraw a second stream 16 from the reaction liquid. The second stream 16 may comprise more than one compound originating from the reaction liquid. At least one of these compounds is advantageously different from the compounds collected in the first permeate by membranes 115 of the first membrane unit. The second stream advantageously comprises reaction (by-)products such as water or the alcohol, which are withdrawn from the reaction liquid 132 in order to shift the reaction to the right hand side and increase product titres. The second stream 16 may be condensed to a liquid phase and may be separated into its separate compounds by an appropriate downstream processing.

Pervaporation membranes 125 may be porous or nonporous membranes allowing a selective mass transfer driven by a partial vapour pressure gradient between substrate and permeate side. In nonporous membranes, a sorption-diffusion-desorption process is sustained through the membrane. Membranes for pervaporation are typically thin film composite membranes, comprising a separation layer defining the membrane characteristics provided on a porous support. Materials for the separation layer useful for pervaporation purposes may be made from a polymer material, a zeolite, a ceramic material or combinations thereof (e.g., polymer membranes with zeolite or ceramic filler particles). The membranes may be either hydrophilic, or organophilic (hydrophobic). Specific examples of useful hydrophilic membranes are made of polyvinyl alcohol, A-type (NaA) or Y-type (NaY) zeolites, or (hybrid) silica. Specific examples of useful hydrophobic membranes are made of polydimethylsiloxane (PDMS) or ZSM-5 (MFI) zeolite.

Hydrophilic pervaporation membranes are advantageously used for withdrawing water from the reaction liquid. For withdrawing the alcohol product from the reaction liquid, organophilic pervaporation membranes may be used. Alternatively, hydrophilic membranes may be used to withdraw the alcohol product.

Membranes used in membrane contactors may be porous or nonporous membranes. In non-porous membranes, a sorption-diffusion-desorption process is sustained through the membrane. Also here, membranes are typically thin film composite membranes. Materials for the separation layer useful for membrane contactor purposes may be made from a polymer material, a zeolite, a ceramic material or combinations thereof.

One advantage of the apparatuses and processes described herein is that they are inherently safer because no associated (external) loops with high cross flows are involved. There is no or less external piping which may be prone to leakage and/or which may expose maintenance operators to (potentially) toxic products. A further advantage is that it has a small footprint, taking much less space in comparison to prior art designs. Yet an additional advantage is that due to the integration of the membrane units 11 and 12 within the reactor, required pumping energy is greatly reduced.

It will be convenient to note that additional membrane units may be integrated in the reactor, similarly to the principles for the first and second membrane units 11, 12 described herein. By way of example, a third membrane unit may be provided in the reactor, either on a separate support frame, or integrated on the support frame of either or both the first and the second membrane units. The support frame of the membranes of the third unit may be pivotally arranged relative to the reactor, or alternatively may be fixed within the reactor. The third membrane unit may be configured for dosing liquid or gaseous compounds to the reaction liquid, through suitable porous or nonporous membranes. Additionally, a fourth, fifth and even further membrane units may be provided.

It will be convenient to note that, although the reactor 13 is described herein as formed of a single vessel (e.g., a single stage reactor), this is no requirement and apparatuses and methods described herein are applicable to multi-stage reactors. A multi-stage reactor typically comprises a plurality, e.g. two, three or even more reactor vessels arranged in series or in cascade. In such cases, the first membrane unit 11 and the second membrane unit 12 need not be arranged in a same reactor vessel, and may be arranged in different ones. Alternatively, it is possible to provide either one, or both the first membrane unit 11 and the second membrane unit 12 in more than one reactor vessel.

## Claims

1. Method of forming an ester or an amide, comprising:
adding a substrate (14) to a reaction liquid (132) comprised in a reactor (13, 33),
reacting the substrate in the reactor to form a product being the ester or the amide,
separating a first stream (15) from the reaction liquid through a first membrane (115), the first stream comprising the product,
separating a second stream (16) from the reaction liquid through a second membrane (125), the second stream comprising a compound distinct from the product, and
withdrawing the first stream and the second stream separately from the reactor,
**characterised in that** the first membrane and the second membrane are moved relative to the reactor during the steps of separating the first stream and separating the second stream.

2. Method of claim 1, wherein the steps of separating the first stream and of separating the second stream are performed in parallel.

3. Method of claim 1 or 2, wherein the first membrane and the second membrane are immersed in the reaction liquid.

4. Method of any one of the preceding claims, wherein separating the first stream comprises filtrating the reaction liquid through the first membrane, wherein the first membrane is a microfiltration, ultrafiltration or nanofiltration membrane.

5. Method of any one of the preceding claims, wherein the compound is water or an alcohol and is separated through the second membrane via pervaporation or liquid-liquid extraction.

6. Method of any one of the preceding claims, wherein the reactor comprises a catalyst, the catalyst being retained by the first membrane.

7. Method of claim 6, wherein the catalyst comprises an enzyme.

8. Method of any one of the preceding claims, wherein the first membrane and the second membrane are rotating or oscillating relative to the reactor during the steps of separating the first stream and separating the second stream.

9. Apparatus for forming an ester or an amide, comprising:
a reactor (13, 33), comprising a reactor vessel (131), a substrate supply (141), a first outlet (111) and a second outlet (121),
a first membrane (115) having a first surface communicating with the reactor vessel and a second surface, opposite the first surface, communicating with the first outlet, and
a second membrane (125) having a third surface communicating with the reactor vessel and a fourth surface, opposite the third surface, communicating with the second outlet,
**characterised in that** the first membrane and the second membrane are moveably arranged relative to the reactor vessel.

10. Apparatus of claim 9, wherein the first membrane and the second membrane are mounted in the reactor vessel such that they are immersed in a reaction liquid (132) when in use.

11. Apparatus of claim 9 or 10, comprising means (112) for applying a first pressure difference between the first surface and the second surface, the first pressure difference being configured to generate a permeate flow from the first surface to the second surface.

12. Apparatus of any one of the claims 9 to 11, comprising means (122) for applying a second pressure difference between the third surface and the fourth surface, the second pressure difference being configured to recover a liquid compound in the reactor vessel through the corresponding vapour at the fourth surface.

13. Apparatus of any one of the claims 9 to 12, wherein the first membrane is a microfiltration, ultrafiltration or nanofiltration membrane, and wherein the second membrane is a membrane for pervaporation or liquid-liquid extraction.

14. Apparatus of claim 13, comprising a support frame (333) pivotally arranged in the reactor vessel (131), wherein the first membrane and the second membrane are attached to the support frame and configured to move integrally with the support frame, and further comprising driving means for rotating or oscillating the support frame relative to the reactor vessel.

15. Apparatus of any one of the claims 9 to 13, comprising one or more frames (113, 123, 313, 323) pivotally arranged in the reactor vessel, wherein the first membrane and the second membrane are attached to a corresponding one of the one or more frames, and further comprising driving means for rotating or oscillating the one or more frames relative to the reactor vessel.
